(19) Europäisches Patentamt
European Patent Office
Office européen des brevets

(11) Veröffentlichungsnummer : **0 337 231 B1**

(12) # EUROPÄISCHE PATENTSCHRIFT

(45) Veröffentlichungstag der Patentschrift :
**17.06.92 Patentblatt 92/25**

(51) Int. Cl.[5] : **A61K 9/10,** A61K 31/47

(21) Anmeldenummer : **89105795.2**

(22) Anmeldetag : **03.04.89**

(54) **i.m. Injektionsformen von Gyrase-Inhibitoren.**

Die Akte enthält technische Angaben, die nach dem Eingang der Anmeldung eingereicht wurden und die nicht in dieserPatentschrift enthalten sind.

(30) Priorität : **15.04.88 DE 3812508**
**25.01.89 DE 3902079**

(43) Veröffentlichungstag der Anmeldung :
**18.10.89 Patentblatt 89/42**

(45) Bekanntmachung des Hinweises auf die Patenterteilung :
**17.06.92 Patentblatt 92/25**

(84) Benannte Vertragsstaaten :
**AT BE CH DE ES FR GB GR IT LI NL SE**

(56) Entgegenhaltungen :
**EP-A- 0 138 018**
**EP-A- 0 210 513**
**EP-A- 0 219 784**
**BE-A- 564 864**

(56) Entgegenhaltungen :
**CHEMICAL & PHARMACEUTICAL BULLETIN, Band 29, Nr. 5, Mai 1981, Seiten 1410-1415, Pharmaceutical Society of Japan, Tokyo,JP; K. HIRANO et al.: "Studies on the absorption of practically waterinsoluble drugs following injection. IV. An approach forpredicting relative intramuscular absorption rates of a drug in oily solution, aqueous suspension and aqueous surfactant solutionin rats"**
**R. Voigt, Lehrbuch der pharm. Technologie, Seite 390/391**
**Europ. Arzneibuch III, Kommentar S. 655/656**

(73) Patentinhaber : **BAYER AG**
**W-5090 Leverkusen 1 Bayerwerk (DE)**

(72) Erfinder : **Pöllinger, Norbert, Dr.**
**Gartenstrasse 17**
**W-5068 Odenthal (DE)**
Erfinder : **Serno, Peter, Dr.**
**Offenbachstrasse 12**
**W-5060 Bergisch Gladbach 2 (DE)**
Erfinder : **Hofmann, Wolfram, Dr.**
**Römerstrasse 338**
**W-5300 Bonn (DE)**
Erfinder : **Beermann, Dieter, Dr.**
**Paul-Ehrlich-Strasse 4**
**W-5600 Wuppertal 1 (DE)**

EP 0 337 231 B1

Anmerkung : Innerhalb von neun Monaten nach der Bekanntmachung des Hinweises auf die Erteilung des europäischen Patents kann jedermann beim Europäischen Patentamt gegen das erteilte europäische Patent Einspruch einlegen. Der Einspruch ist schriftlich einzureichen und zu begründen. Er gilt erst als eingelegt, wenn die Einspruchsgebühr entrichtet worden ist (Art. 99(1) Europäisches Patentübereinkommen).

## Beschreibung

Die Erfindung betrifft i.m. Injektionsformen, die als Wirkstoff Gyrase-Inhibitoren aus der Gruppe der Chinolon- und 1,8-Naphthyridon-3-carbonsäuren enthalten, deren Herstellung und deren Verwendung als Arzneimittel.

Bisher sind Tabletten zur peroralen Applikation sowie größervolumige Infusionslösungen (0,2 %/ 50, 100 ml) bzw. Infusionskonzentrate (1 % / 10 ml), die beispielsweise Ciprofloxacin als wirksame Substanz enthalten, verfügbar. Dagegen ist bis heute keine zufriedenstellende Formulierung für die i.m. Applikation entwickelt worden. So sind Lösungen von beispielsweise Ciprofloxacin bis 5 %, die intramuskulär appliziert werden sollen, aufgrund ihrer unphysiologischen pH-Werte im sauren bzw. alkalischen Bereich sehr schlecht verträglich. Nach der i.m. Injektion wäßriger saurer und alkalischer Lösungen wurden im Muskelgewebe erhebliche Unverträglichkeiten wie etwa Nekrosen festgestellt.

Derartige unerwünschte Nebenwirkungen können auch bei Formulierungen festgestellt werden, bei denen es sich um Lösungen der Wirkstoffe in Ölen oder um Öl-in-Wasser- oder Wasser-in-Öl-Emulsionen von Wirkstoffzubereitungen handelt, wie sie aus der EP-A-0 210 513 bekannt geworden sind.

Überraschenderweise konnte gefunden werden, daß Ciprofloxacin nach intramuskulärer Applikation dann gut verträglich ist, wenn es in.

Form einer öligen Suspension des Betains oder dessen Salzes verabreicht wird. Weiterhin konnte überraschenderweise festgestellt werden, daß die öligen Suspensionen, die Ciprofloxacin in wasserlöslicher Form enthalten, etwa in Form des Hydrochlorids, Laktates, Mesilates, Methansulfonates und anderer Salze, den Wirkstoff sehr rasch freizusetzen imstande sind, insbesondere dann, wenn die Benetzbartkeit des öligen Trägermediums durch Zusatz grenzflächenaktiver Stoffe erhöht wird.

Demgegenüber gewährleisten wäßrige Suspensionen, die den Wirkstoff in der Betain-Form enthalten, eine protrahierte Freisetzung des Wirkstoffs.

Die Steuerung und Kontrolle der Freisetzung ist möglich über die Wahl der Teilchengröße des Wirkstoffs bzw. die Hilfsstoffzusammensetzung.

Die Erfindung betrifft i.m. Injektionsformulierungen von Gyrase-Inhibitoren, enthaltend 0,05 bis 70 Gew.-% eines Gyrase-Inhibitors der allgemeinen Formel

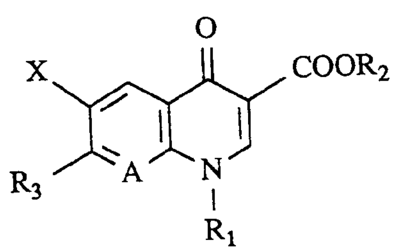

(I)

in welcher

$R^1$ für Methyl, Ethyl, Propyl, Isopropyl, Cyclopropyl, Vinyl, 2-Hydroxyethyl, 2-Fluorethyl, Methoxy, Amino, Methylamino, Dimethylamino, Ethylamino, Phenyl, 4-Fluorphenyl, 2,4-Difluorphenyl,

$R^2$ für Wasserstoff, Alkyl mit 1 bis 4 Kohlenstoffatomen (5-Methyl-2-oxo-1,3-dioxol-4-yl)-methyl,

$R^3$ für Methyl oder eine cyclische Aminogruppe wie

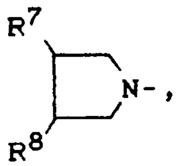 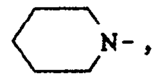 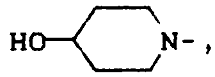 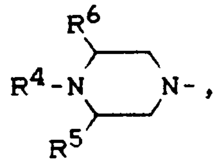

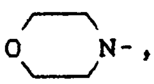 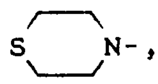 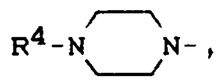 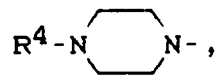

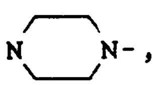 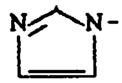 , 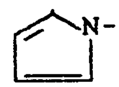

steht, worin

$R^4$ für Wasserstoff, Alkyl mit 1 bis 4 Kohlenstoffatomen, 2-Hydroxyethyl, Allyl, Propargyl, 2-Oxopropyl, 3-Oxobutyl, Phenacyl, Formyl, $CFCl_2$-S-, $CFCl_2$-$SO_2$-, $CH_3O$-CO-S-, Benzyl, 4-Aminobenzyl,

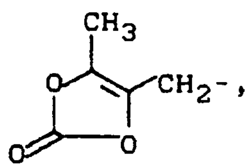

$R^5$ für Wasserstoff, Methyl,

$R^6$ für Wasserstoff, Alkyl mit 1 bis 4 Kohlenstoffatomen, Phenyl, Benzyloxymethyl,

$R^7$ für Wasserstoff, Amino, Methylamino, Ethylamino, Aminomthyl, Methylaminomethyl, Ethylaminomethyl, Dimethylaminomethyl, Hydroxy, Hydroxymethyl,

$R^8$ für Wasserstoff, Methyl, Ethyl oder Chlor steht,

X für Fluor, Chlor oder Nitro und

A für N oder C-$R^6$ steht, worin

$R^6$ für Wasserstoff, Halogen wie Fluor oder Chlor, Methyl oder Nitro steht oder auch gemeinsam mit $R^1$ eine Brücke der Struktur

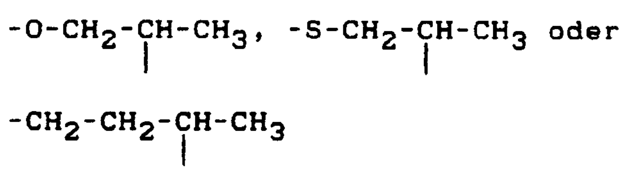
oder

bilden kann,

gegebenenfalls als Salz mit einer Säure oder Base in öliger Suspension.

Mit den Gyrase-Inhibitoren enthaltenden i.m. Injektionen kann nicht nur eine systemische Wirkung, die je nach Zusammensetzung der Formulierung zeitlich steuerbar ist, erzielt werden. Man kann daneben auch lokale Infektionsherde auf direktem Wege erreichen und gezielt über längere Zeiträume behandeln.

Die Gyrase-Inhibitoren können in den erfindungsgemäßen Injektionsformen als solche oder als Salz mit einer Säure oder Base angewendet werden. Auch eine Verwendung als Prodrug, beispielsweise in Form von Estern, ist möglich.

Die erfindungsgemäßen Zubereitungen enthalten 0,05-70 Gew.-%, bevorzugt 2,5-50 Gew.-% des Wirkstoffs.

Besonders bevorzugt enthalten die erfindungsgemäßen i.m. zu injizierenden Suspensionen 10-60 % G/G Wirkstoff der obigen Formel.

Insbesondere sind Ciprofloxacin, Norfloxacin, Pefloxacin, Amifloxacin, Pirfloxacin, Ofloxacin, Fleroxacin, Lomefloxacin und/oder Enoxacin in den genannten Zubereitungen enthalten. Ganz besonders bevorzugt enthalten die erfindungsgemäßen Formulierungen auch die Wirkstoffe der europäischen Patentanmeldungen mit den Publikationsnummern 153 163, 106 489, 153 828, 195 316, 167 763 oder 126 355.
Insbesondere sind folgende Wirkstoffe zu nennen:
6-Chlor-7-[3-(4-chlorphenyl)-1-piperazinyl]-1-cyclopropyl-8-fluor-1,4-dihydro-4-oxo-3-chinolincarbonsäure,
6-Chlor-1-cyclopropyl-8-fluor-7-[3-(4-fluorphenyl)-1-piperazinyl]-1,4-dihydro-4-oxo-3-chinolincarbonsäure,
7-[3-(4-Bromphenyl)-1-piperazinyl]-6-chlor-1-cyclopropyl-8-fluor-1,4-dihydro-4-oxo-3-chinolincarbonsäure,
6-Chlor-1-cyclopropyl-8-fluor-1,4-dihydro-7-[3-(4-methylphenyl)-1-piperazinyl]-4-oxo-3-chinolincarbonsäure,
7-[3-(4-Biphenylyl)-1-piperazinyl]-6-chlor-1-cyclopropyl-8-fluor-1,4-dihydro-4-oxo-3-chinolincarbonsäure,
6-Chlor-1-cyclopropyl-8-flor-1,4-dihydro-7-[3-(4-methoxyphenyl)-1-piperazinyl]-4-oxo-3-chinolincarbonsäure,
6-Chlor-1-cyclopropyl-8-fluor-1,4-dihydro-7-[3-(4-hydroxyphenyl)-1-piperazinyl]-4-oxo-3-chinolincarbonsäure,
8-Chlor-1-cyclopropyl-6-fluor-1,4-dihydro-4-oxo-7-(3-phenyl-1-piperazinyl)-3-chinolincarbonsäure,
8-Chlor-1-cyclopropyl-6-fluor-1,4-dihydro-4-oxo-7-[(4-nitrophenyl)-1-piperazinyl]-3-chinolincarbonsäure,
8-Chlor-1-cyclopropyl-6-fluor-1,4-dihdyro-4-oxo-7-[3-(4-piperidinophenyl)-1-piperazinyl]-3-chinolincarbonsäure,
8-Chlor-1-cyclopropyl-6-fluor-1,4-dihydro-4-oxo-8-[3-(3,4-dimethoxyphenyl)-1-piperazinyl]-3-chinolincarbonsäure,
8-Chlor-1-cyclopropyl-6-fluor-1,4-dihydro-4-oxo-7-[3-(3,4,5-trimethoxyphenyl)-1-piperazinyl]-3-chinolincarbonsäure,
8-Chlor-1-cyclopropyl-6-fluor-1,4,dihydro-4-oxo-7-[3-(2-thienyl)-1-piperazinyl]-3-chinolincarbonsäure,
8-Chlor-1-cyclopropyl,6-fluor-1,4-dihydro-4-oxo-7-piperidino-3-chinolincarbonsäure,
7-(3-Amino-1-pyrrolidinyl)-8-chlor-1-cyclopropyl-6-fluor-1,4-dihydro-4-oxo-3-chinolincarbonsäure (Wirkstoff B),
6,8-Dichlor-1-cyclopropyl-1,4-dihydro-4-oxo-7-(1-piperazinyl)-3-chinolincarbonsäure,
7-(4-Acetyl-1-piperazinyl)-6,8-dichlor-1-cyclopropyl-1,4-dihydro-4-oxo-3-chinolincarbonsäure,
7-(4-Acetyl-1-piperazinyl)-6-chlor-1-cyclopropyl-8-fluor-1,4-dihydro-4-oxo-3-chinolincarbonsure,
6-Chlor-1-cyclopropyl-8-fluor-1,4-dihydro-7-(4-isopropyl-1-piperazinyl)-4-oxo-3-chinolincarbonsäure,
6-Chlor-1-cyclopropyl-8-fluor-1,4-dihydro-4-oxo-7-morpholino-3-chinolincarbonsäure,
6-Chlor-1-cyclopropyl-8-fluor-1,4-dihydro-4-oxo-7-thiomorpholino-3-chinolincarbonsäure,
8-Chlor-1-cyclopropyl-7-(4-ethyl-3-oxo-1-piperazinyl)-6-fluor-1,4-dihydro-4-oxo-3-chinolincarbonsäure,
1-Cyclopropyl-6,8-difluor-1,4-dihydro-4-oxo-7-(1-piperazinyl)-3-chinolincarbonsäure,
1-Cyclopropyl-6,8-difluor-1,4-dihydro-4-oxo-7-(4-methyl-1-piperazinyl)-3-chinolincarbonsäure,
1-Cyclopropyl-6,8-difluor-1,4-dihydro-4-oxo-7-(4-ethyl-1-piperazinyl)-3-chinolincarbonsäure,
1-Cyclopropyl-6,8-difluor-1,4-dihydro-4-oxo-7-(3-methyl-1-piperazinyl)-3-chinolincarbonsäure,
1-Cyclopropyl-6,8-difluor-1,4-dihydro-4-oxo-7-(3,4-dimethyl-1-piperazinyl)-3-chinolincarbonsäure
1-Cyclopropyl-6,
8-difluor-1,4-dihydro-4-oxo-7-(4-ethyl-3-methyl-1-piperazinyl)-3-chinolincarbonsäure,
1-Cyclopropyl-6,8-difluor-1,4-dihydro-4-oxo-7-[4-(2-hydroxyethyl)-3-methyl-1-piperazinyl]-3-chinolincarbonsäure,
1-Cyclopropyl-6,8-difluor,1,4-dihydro-4-oxo-7-[4-(3-hydroxypropyl)-3-methyl-1-piperazinyl]-3-chinolincarbonsäure,
1-Cyclopropyl-6,8-difluor-1,4-dihydro-4-oxo-7-(2,5-dimethyl-1-piperazinyl)-3-chinolincarbonsäure,
1-Cyclopropyl-6,8-difluor-1,4-dihydro-4-oxo-7-(4-ethyl-2,5-dimethyl-1-piperazinyl)-3-chinolincarbonsäure,
1-Cyclopropyl-6,8-difluor-1,4-dihydro-4-oxo-7-(3,5-dimethyl-1-piperazinyl)-3-chinolincarbonsäure (Wirkstoff A),
1-Cyclopropyl-6,8-difluor-1,4-dihydro-4-oxo-7-(3,4,5-trimethyl-1-piperazinyl)-3-chinolincarbonsäure,
1-Cyclopropyl-6,8-difluor-1,4-dihydro-4-oxo-7-(4-ethyl-3,5-dimethyl-1-piperazinyl)-3-chinolincarbonsäure,
1-Cyclopropyl-6,8-difluor-1,4-dihydro-4-oxo-7-(3-ethyl-1-piperazinyl)-3-chinolincarbonsäure,
1-Cyclopropyl-6,8-difluor-1,4-dihydro-4-oxo-7-(3-n-propyl-1-piperazinyl)-3-chinolincarbonsäure,
1-Cyclopropyl-6,8-difluor-1,4-dihydro-4-oxo-7-(3-aminopyrrolidinyl-3-chinolincarbonsäure (Wirkstoff C),
1-Cyclopropyl-6,8-difluor-1,4-dihydro-4-oxo-7-(3-isopropyl-1-piperazinyl)-3-chinolincarbonsäure,
1-Cyclopropyl-6,8-difluor-1,4-dihydro-4-oxo-7-(3-isobutyl-1-piperazinyl)-3-chinolincarbonsäure,
1-Cyclopropyl-6,8-difluor-1,4-dihydro-4-oxo-(3-methyl-4-n-propyl-1-piperazinyl)-3-chinolincarbonsäure,
1-Cyclopropyl-6,8-difluor-1,4-dihydro-4-oxo-7-(3-methyl-4-isopropyl)-1-piperazinyl)-3-chinolincarbonsäure,
1-CyclopropyL-6,8-difluor-1,4-dihydro-4-oxo-7-(4-n-butyl-3-methyl-1-piperazinyl)-3-chinolincarbonsäure,
1-Cyclopropyl-6,8-difluor-1,4-dihydro-4-oxo-7-morpholinyl-3-chinolincarbonsäure,
und deren pharmazeutisch verwendbaren Säureadditionssalze, Alkalisalze, Erdalkalisalz oder Hydrate.

Besonders bevorzugt werden schließlich auch Ciprofloxacin oder Enrofloxacin als Wirkstoff in den erfindungsgemäßen Formulierungen eingesetzt.

Unter den im folgenden geschilderten Voraussetzungen sind intramuskuläre Injektionsformen auf öliger Basis, die die Wirksubstanz in wasserlöslicher, kristalliner oder amorpher Form enthalten, z.B. in Form des Hydrochlorids, Laktates, Mesilates, p-Tolylsulfonates und anderer Salze, hergestellt mit physiologisch verträglichen Säuren, sowie intramuskuläre Injektionsformen auf öliger Basis, die grenzflächenaktive Substanzen, wie

z.B. Lecithin in Form von Soja-, Ei-, Cerebral- oder Raps-Lecithin oder andere physiologisch verträgliche Tenside in Konzentrationen von 0,1-30 %, inbesondere von 0,2-10 %, ganz besonders von 0,5-5 % G/V enthalten, ebenso wie intramuskuläre Injektionsformen auf öliger Basis, die neben den wasserlöslichen, in Form ihrer Salze eingesetzten Wirkstoffen einen Überschuß an pysiologisch verträglicher Säure wie z.B. Milchsäure oder Citronensäure im Bereich von 1-300 mmol/l, insbesondere 5-50 mmol/l, vorzugsweise 10-30 mmol/l enthalten, besonders bevorzugte Gegenstände der Erfindung.

Ölige Suspension mit Gyrase-Hemmern vom Chinolon-Typ enthalten den Wirkstoff entweder in der Betain-Form oder in Form wasserlöslicher Salze. Zur Salzbildung geeignete physiologisch verträgliche Säuren wurden vorstehend beispielhaft genannt.

Ölige Suspensionen können als nichtwäßrige Trägerstoffe beispielsweise Mandelöl, Arachisöl, Olivenöl, Mohnöl, Sesamöl, Baumwollsamenöl, Sojabohnenöl, Maisöl, Rizinusöl, Ethylolelat, Oleyloleat, Isopropylmyristat, Isopropylpalmitat, mittelkettige Triglyceride u.a. enthalten. Als weitere, mit den genannten Substanzen kombinbierbare Hilfsstoffe können Ethanol, Glycerol, Propylenglykol, Polyethyleglykol, 1,3-Butandiol, Benzylalkohol, Diethylenglykol und Triethylenglykol unterschiedlicher Provenienz, Polyoxyethylen - Polyoxypropylen -Copolymerisate vom Typ des Pluronic®, Polyoxysorbitanfettsäureester, Sorbitanfettsäureester, Monoolein, Cremophor EL®, Inwitor 742® und verschiedene Lecithintypen wie Sojabohnenlecithin, Eilecithin, Cerebrallecithin und Rapslecithin verwendet werden. Als Antioxidantien finden Verwendung $\alpha$-, $\beta$-, $\gamma$-, $\delta$- Tocopherol, Ascorbylpalmitat, Ascorbylstearat, L-Cystein, Thiodipropionsäure, Thiomilchsäure, Thioglycolsäure, Monothioglycerol, Propylgallat, Butylhydroxyanisol, Butylhydroxytoluol u.a.

Gegebenenfalls kann eine gewünschte Viskosität durch Verdünnungsmittel wie Ethanol oder Benzylalkohol sowie durch Verdickungsmittel wie Aluminiumstearat herbeigeführt werden.

Säuren können als Absorptionsverstärker zugesetzt werden. Geeignete Säuren wurden vorstehend beispielhaft genannt.

Viskositäts-Werte für ölige Suspensionen sind 5-500 mPa.s, vorzugsweise 10-150 mPa.s.

Die Herstellung öliger Suspensionen erfolgt in der Weise, daß der ölige Träger mit darin enthaltenen Hilfsstoffen und der auf gewünschte Teilchengröße zerkleinerte Wirkstoff mit geeigneten Geräten (s.o.) miteinander vereingt und homogenisiert werden. Die Partikelgröße von 90 % der Partikel beträgt 0,5-150 µm bevorzugt 4-12 µm. Falls sich eine Endsterillisation im Abgabegefäß wegen möglicher Teilchengrößenveränderung des Wirkstoffes verbietet, hat die Herstellung der Suspension wiederum unter aseptischen Kautelen zu erfolgen. Eine Sterilfiltration der geeignete Hilfsstoffe in gelöster Form enthaltenden öligen Phase ist ebenso angezeigt wie eine antimikrobielle Vorbehandlung des Wirkstoffes, etwa durch Hitzebehandlung. Eine Endsterilisation in Form einer U-Strahlen-Sterilisation kann ebenfalls angewandt werden.

Neben einer Fertigsuspension kann auch eine kurz vor der Applikation frisch zuzubereitende Formulierung angeboten werden. Der Wirkstoff muß sich dabei in kurzer Zeit durch Umschütteln des die Formulierung enthaltenden Gefäßes homogen in flüssigen Träger suspendieren lassen.

Außerdem gehören Suspensionskonzentrate, die kurz vor der Applikation in die erfindungsgemäßen Zubereitungen überführt werden, zur Erfindung.

Diese Konzentrate können verschiedene Zusammensetzungen haben. Sämtliche weitere Kombinationen von Konzentraten und/oder Suspensionen und zur Verdünnung erforderlichen Lösungsmitteln bzw. Lösungen, die zu den erfindungsgemäßen Suspensionen führen, sind Gegenstand dieser Erfindung.

Auch sonstige Darreichungsformen oder Kombinationen von Darreichungsformen, die letztendlich zu den erfindungsgemäßen Injektionslösungen führen - und dies unabhängig von der Vorgehensweise - sind Gegenstand dieser Erfindung.

Die Behältnisse, in denen Suspensionen, Wirkstoff, Lösungsmittel und sonstige Darreichungsformen wie Suspensionskonzentrate abgefüllt werden, können aus Glas wie auch aus Kunststoff bestehen. Dabei können die Behältermaterialien Substanzen enthalten, die dem Inhalt einen besondere Schutz verleihen, wie z.B. einen Lichtschutz oder Sauerstoffschutz. Neben kleinvolumigen Gefäßen, aus denen die Suspension vor der Applikation in die Injektionsspritze aufgezogen werden muß, kann es sich dabei auch um Fertiginjektionssysteme handeln.

Die erfindungsgemäßen Injektionsformulierungen finden Verwendung bei der therapeutischen Behandlung des menschlichen oder tierischen Körpers.

Die erfindungsgemäßen Formulierungen zeigen bei geringer Toxizität ein breites antibakterielles Spektrum gegen gram-positive und gram-negative Keime, insbesondere gegen Enterobakteriaceen; vor allem auch gegen solche, die resistent sind gegen verschiedene Antibiotika, wie z.B. Penicilline, Cephalosporine, Aminnoglykoside, Sulfonamide, Tetracycline.

Die erfindungsgemäßen Formulierungen sind gegen ein sehr breites Spektrum von Mikroorganismen wirksam. Mit ihrer Hilfe können gram-negative und gram-positive Bakterien und bakterienähnliche Mikroorganismen bekämpft sowie die durch diese Erreger hervorgerufenen Erkrankungen verhindert, gebessert und/oder

geheilt werden.

Besonders wirksam sind die erfindungsgemäßen Formulierungen gegen Bakterien und bakterienähnliche Mikroorganismen. Sie sind daher besonders gut zur Prophylaxe und Chemotherapie von lokalen und systemischen Infektionen in der Human- und Tiermedizin geeignet, die durch diese Erreger hervorgerufen werden.

Beispielsweise können lokale und/oder systemische Erkrankungen behandelt und/oder verhindert werden, die durch die folgenden Erregar oder durch Mischungen der folgenden Erreger verursacht werden: Gram-positive Kokken, z.B. Staphylokokken (Staph. aureus, Staph. epidermidis) und Streptokokken (Strept. agalactiae, Strept. faecalis, Strept. pneumoniae, Strept. pyogenes); gram-negative Kokken (Neisseria gonorrhoeae) sowie gram-negative Stäbchen wie Enterobakteriaceen, z.B. Escherichia coli, Haemophilus influenzae, Citrobacter (Citrob. freundii, Citrob. divernis), Salmonella und Shigella; ferner Klebsiellen (Klebs. pneumoniae, Klebs. oxytoca), Enterobacter (Ent. aerogenes, Ent. agglomerans), Hafnia, Serratia (Serr. marcescens), Proteus (Pr. mirabilis, Pr. rettgeri, Pr. vulgaris), Providencia, Yersinia, sowie die Gattung Acinetobacter. Darüber hinaus umfaßt das antibakterielle Spektrum die Gattung Pseudomonas (Ps. aeruginosa, Ps. maltophilia) sowie strikt anaerobe Bakterien wie z.B. Bacteroides fragilis, Vertreter der Gattung Peptococcus, Peptostreptococcus sowie die Gattung Clostridium; ferner Mykoplasmen (M. pneumoniae, M. hominis, M. urealyticum) sowie Mykobakterien, :.B. Mycobacterium tuberculosis.

Die obige Aufzählung von Erregern ist lediglich beispielhaft und keineswegs beschränkend aufzufassen. Als Krankheiten, die durch die genannten Erreger oder Mischinfektionen verursacht und durch die erfindungsgemäßen Verbindungen verhindert, gebessert oder geheilt werden können, seien beispielsweise genannt:

Infektionskrankheiten beim Menschen wie zum Beispiel Otitis, Pharyngitis, Pneumonie, Peritonitis, Pyelonephritis, Cystitis, Endocarditis, Systeminfektionen, Bronchitis (akut, chronisch), septische Infektionen, Erkrankungen der oberen Luftwege, diffuse Panbronchiolitis, pulmonäres Emphysem, Dysenterie, Enteritis, Leberabszesse, Urethritis, Prostatitis, Epididymitis, gastrointestinale Infektionen, Knochen- und Gelenkinfektionen, zystische Fibrose, Hautinfektionen, postoperative Wundinfektionen, Abszesse, Phlegmone, Wundinfektionen, infizierte Verbrennungen, Brandwunden, Infektionen im Mundbereich, Infektionen nach Zahnoperationen, Osteomyelitis, septische Arthritis, Cholecystitis, Peritonitis mit Appendicitis, Cholangitis, intraabdominale Abszesse, Pankreatitis, Sinusitis, Mastoiditis, Mastitis, Tonsillitis, Typhus, Meningitis und Infektionen des Nervensystems, Salpingitis, Endometritis, Genital-Infektionen, Pelveoperitonitis und Augeninfektionen.

Außer beim Menschen können bakterielle Infektionen auch bei anderen Spezies behandelt werden. Beispielhaft seien genannt:

Schwein: Coli-diarrhoe, Enterotoxämie, Sepsis, Dysenterie, Salmonellose, Mastitis-Metritis-Agalaktie-Syndrom, Mastitis;

Wiederkäuer (Rind, Schaf, Ziege): Diarrhoe, Sepsis, Bronchopneumonie, Salmonellose, Pasteurellose, Mykoplasmose, Genitalinfektionen;

Pferd: Bronchopneumonien, Fohlenlähme, puerperale und postpuerperale Infektionen, Salmonellose;

Hund und Katze: Bronchopneumonie, Diarrhoe, Dermatitis, Otitis, Harnwegsinfekte, Prostatitis;

Geflügel (Huhn, Pute, Wachtel, Taube, Ziervögel und andere): Mycoplasmose, E. coli-Infektionen, chronische Luftwegserkrankungen, Salmonellose, Pasteurellose, Psittakose.

Ebenso können bakterielle Erkrankungen bei der Aufzucht und Haltung von Nutz- und Zierfischen behandelt werden, wobei sich das antibakterielle Spektrum über die vorher genannten Erreger hinaus auf weitere Erreger wie zum Beispiel Pasteurella, Brucella, Campylobacter, Listeria, Erysipelothrix, Corynebakterien, Borrelia, Treponema, Nocardia, Rikettsien, Yersinia erweitert.

Die in den Beispielen benannten Hilfsstoffe sind im Handel erhältlich und zum Teil in H.P. Fiedler, Lexikon der Hilfsstoffe für Pharmazie, Kosmetik und angrenzende Gebiete, Editio Cantor KG - Aulendorf i. Württ. 1971 (z.B. Tween®, Miglyol®) definiert.

Die Zeichnungen Fig. 1 und Fig. 2 zeigen Vergleichsversuche, die die gute Verträglichkeit einer Ciprofloxacin-i.m.-Suspension im Gegensatz zu Ciprofloxacin-i.m.-Lösungen demonstrieren.

Fig. 1 zeigt den Verlauf der Kreatininkinase nach i.m.-Applikation von Ciprofloxacin-i.m.-Lösungen, einer i.m.-Suspension sowie von Vergleichslösungen.

Fig. 2 zeigt die lokale Reizung nach i.m.-Applikation von Ciprofloxacin-Lösungen sowie einer Ciprofloxacin-i.m.-Suspension und Vergleichslösungen.

Fig. 3 zeigt die Blutplasmaspiegel nach Anwendung einer öligen Ciprofloxacin-HCl-Suspension (Nr. 311) an Kaninchen.

Die sehr schnelle Absorption des Ciprofloxacin-HCl aus einer öligen Suspension ( Nr. 311) in das Blut ist klar ersichtlich.

Ergebnis:

1) Die lokalen Reizungen nach i.m. Applikation von Ciprofloxacin-Suspension 5 % G/V (Nr. 311, 918) liegen deutlich unter denen, die durch i.m.-Lösungen (Nr. 401-H, 904) erzeugt werden. (Kaninchentest)

2) Im Gegensatz zu den geprüften i.m.-Lösungen zieht die Applikation der Ciprofloxacin-Suspension keinen Anstieg der Serum-Kreatininkinase nach sich.

Im folgenden (Kaninchentest) werden die für die Versuche benutzten Formulierungen beschrieben.

Ciprofloxacin intramuscular injection (E-No. 401-H) solution 5 % W/V

| | |
|---|---|
| Ciprofloxacin | 50,0 g |
| Milchsäure verseift 20 % | 131,0 g |
| Wasser für Injektionszwecke | 840,6 g |
| | 1021,6 g |

klare, gelbliche Lösung; pH 3,9; isoton

Ciprofloxacin i.m. Injektionslösung (E-Nr. 904) 5 % G/V 2 ml

| | |
|---|---|
| Ciprofloxacin | 50,0 g |
| Essigsäure 100 % | 25,0 g |
| Wasser für Injektionszwecke | 943,7 g |
| | 1018,7 g (1 l) |

klare, gelbe Lösung; pH 4,2

Ciprofloxacin i.m. Injektionslösung (E-Nr. 902)
5 % G/V 2 ml

| | | |
|---|---|---|
| Ciprofloxacin | | 50,0 g |
| Methansulfonsäure | | 14,5 g |
| Glycerin wasserfrei | | 12,5 g |
| 0,1 n NaOH-Lösung | ad pH | 4,2 |
| Wasser f. Injektionszwecke | | 945,5 g |
| | | 1022,5 g (1 l) |

klare, gelbe Lösung; pH 4,2

Ciprofloxacin i.m. Suspension (E-Nr. 918)
5 % G/V 2 ml

| | |
|---|---|
| Ciprofloxacin | 50,0 g |
| 20 % G/G NaOH-Lösung | 55,2 g |
| Citronensäure Feingrieß | 17,9 g |
| Tylopur C 300 P | 2,0 g |
| Wasser f. Injektionszwecke | 907,3 g |
| | 1032,4 g (1 l) |

absetzende, wiederaufschüttelbare Suspensionen pH 7; Teilchengröße der Kristalle überwiegend kleiner 10 µm.

| Ciprofloxacin Suspension 5 % W/V | (E-Nr. 311) |
|---|---|
| Ciprofloxacin-HCl | 58,2 g |
| Phosphotipon 100 | 5,0 g |
| Benzylalkohol destilliert | 20,0 g |
| Miglyol 812® | 883,8 g |
| | 967,0 g |

ölige Suspension,

| Ciprofloxacin Placebo | (E-Nr. 009 H) |
|---|---|
| Milchsäure verseift 20 % | 64,06 g |
| 2 N NaOH-Lösung | 38,4 g |
| Natriumchlorid | 1,46 g |
| Wasser f. Injektionszwecke | 900,08 g |
| | 1004,0 g |

klare, farblose Lösung; isoton; pH 3,9

Ciprofloxacin Plac. Nr. 954

Ciprofloxacin i.m. Injektionslösung Placebo 5 % G/V 2 ml

| | |
|---|---|
| Essigsäure 100 % | 50,0 g |
| 1 n NaOH-Lösung | ad pH 4,2 |
| Wasser f. Injektionszwecke | 956,7 g |
| | 1006,7 g |

klare, farblose Lösung; pH etwa 4,2

Ciprofloxacin Plac. Nr. 952

Ciprofloxacin i.m. Injektionslösung Placebo
5 % G/V 2 ml

| | |
|---|---|
| Methansulfonsäure | 14,5 g |
| Glycerol wasserfrei | 12,5 g |
| 1 n NaOH-Lösung | ca. 158,06 g |
| Wasser f. Injektionszwecke | 825,54 g |
| | 1010,6 g |

klare, nahezu farblose Lösung;
pH etwa 4,2
Osmolalität ca. 425 m Osmol

Ciprofloxacin Plac. Nr. 968

Ciprofloxacin i.m. Injektionslösung Placebo
5 % G/V 2 ml

| | |
|---|---|
| Citronensäure Feingrieß | 17,9 g |
| 20 % G/G NaOH-Lösung | 55,6 g |
| Tylopur C 300 P® | 2,0 g |
| Wasser f. Injektionszwecke | 940,3 g |
| | 1015,8 g (1 l) |

farblose, leicht opalesz. Lösung; pH 6,8

Beispiele:

1.

| | | | |
|---|---|---|---|
| Ciprofloxacin-Hydrochlorid | 5 | g | Suspension von Ciprofloxacin-Hydrochlorid |
| Ethyloleat | 50 | g | |

2.

| | | |
|---|---|---|
| Ciprofloxacin-Hydrochlorid | 10 | g |
| Ethyloleat | 50 | g |

3.

| | | |
|---|---|---|
| Ciprofloxacin-Hydrochlorid | 30 | g |
| Ethyloleat | 50 | g |

4.

| | | |
|---|---|---|
| Ciprofloxacin-Hydrochlorid | 25 | g |
| Ethyloleat | 50 | g |

5.

| | | |
|---|---|---|
| Ciprofloxacin-Hydrochlorid | 5 | g |
| Miglyol 812® | 50 | g |

6.

| | | |
|---|---|---|
| Ciprofloxacin-Hydrochlorid | 10 | g |
| Miglyol 812® | 50 | g |

7.

| | | |
|---|---|---|
| Ciprofloxacin-Hydrochlorid | 15 | g |
| Miglyol 812® | 50 | g |

8.

| | | |
|---|---|---|
| Ciprofloxacin-Hydrochlorid | 5 | g |
| Erdnußöl | 50 | g |

9.

| | | |
|---|---|---|
| Ciprofloxacin-Hydrochlorid | 5 | g |
| Ethyloleat/Phospholipon 100® = 99,5/0,5 | 50 | g |

10.

| | | |
|---|---|---|
| Ciprofloxacin-Hydrochlorid | 30 | g |
| Ethyloleat/Phospholipon 100® = 99,5/0,5 | 50 | g |

11.

| | | |
|---|---|---|
| Ciprofloxacin-Hydrochlorid | 10 | g |
| Migloyl 812®/Phospholipon 100® = 99,5/0,5 | 50 | g |

12.

| | | |
|---|---|---|
| Ciprofloxacin-Hydrochlorid | 15 | g |
| Migloyl 812®/Phospholipon 100® = 99,5/0,5 | 50 | g |

13.

| | | |
|---|---|---|
| Ciprofloxacin | 30 | g |
| Ethyloleat® | 50 | g |

14.

| | | |
|---|---|---|
| Ciprofloxacin | 10 | g |
| Miglyol 812® | 50 | g |

15.

| | | |
|---|---|---|
| Ciprofloxacin | 2,5 | g |
| Erdnußöl | 50 | g |

16.

| | | |
|---|---|---|
| Ciprofloxacin-Hydrochlorid | 30 | g |
| Ethyloleat | 50 | g |
| Ascorbylpalmitat | 0,1 | g |
| Lecithin (Phospholipon 100)® | 0,5 | g |

17.

| | | |
|---|---|---|
| Ciprofloxacin-Hydrochlorid | 25 | g |
| Miglyol 812 | 50 | g |
| Ascorbylpalmitat | 0,1 | g |
| Lecithin (Phospholipon 100)® | 0,5 | g |

18.

| | | |
|---|---|---|
| Ciprofloxacin-Hydrochlorid | 30 | g |
| Ethyloleat | 50 | g |
| Ascorbylpalmitat | 0,1 | g |
| Lecithin (Phospholipon 100)® | 0,5 | g |
| Benzylalkohol | 3 | g |

19.

| | | |
|---|---|---|
| Ciprofloxacin-Hydrochlorid | 25 | g |
| Miglyol 812 | 50 | g |
| Benzylalkohol | 3 | g |
| Lecithin (Phospholipon 100)® | 0,5 | g |
| Ascorbylpalmitat | 0,1 | g |

| | | | |
|---|---|---|---|
| 20. | | | |
| Norfloxacin | 5 | g | Suspension von Norfloxacin in öligem Träger |
| 21. | | | |
| Ofloxacin | 10 | g | |
| Ethyloleat | 50 | g | |
| 22. | | | |
| Norfloxacin | 30 | g | |
| Ethyloleat | 50 | g | |
| 23. | | | |
| Norfloxacin | 25 | g | |
| Ethyloleat | 50 | g | |
| 24. | | | |
| Ofloxacin | 5 | g | |
| Miglyol 812® | 50 | g | |
| 25. | | | |
| Pefloxacin | 10 | g | |
| Miglyol 812® | 50 | g | |
| 26. | | | |
| Wirkstoff A | 15 | g | |
| Miglyol 812® | 50 | g | |
| 27. | | | |
| Wirkstoff | 5 | g | |
| Erdnußöl | 50 | g | |
| 28. | | | |
| Wirkstoff B | 5 | g | |
| Ethyloleat/Phospholipon 100® = 99,5/0,5 | 50 | g | |

29.

| | | |
|---|---|---|
| Wirkstoff C | 30 | g |
| Ethyloleat/Phospholipon 100® = 99,5/0,5 | 50 | g |

30.

| | | |
|---|---|---|
| Wirkstoff B | 10 | g |
| Miglyol 812®/Phospholipon 100® = 99,5/0,5 | 50 | g |

31.

| | | |
|---|---|---|
| Wirkstoff A | 15 | g |
| Miglyol 812®/Phospholipon 100® = 99,5/0,5 | 50 | g |

32.

| | | |
|---|---|---|
| Wirkstoff C | 30 | g |
| Ethyloleat | 50 | g |

33.

| | | |
|---|---|---|
| Wirkstoff A | 10 | g |
| Miglyol 812® | 50 | g |

34.

| | | |
|---|---|---|
| Wirkstoff B | 2,5 | g |
| Erdnußöl | 50 | g |

35.

| | | |
|---|---|---|
| Wirkstoff B | 30 | g |
| Ethyloleat | 50 | g |
| Ascorbylpalmitat | 0,1 | g |
| Lecithin (Phospholipon 100®) | 0,5 | g |

36.

| | | |
|---|---|---|
| Wirkstoff C | 25 | g |
| Miglyol 812® | 50 | g |
| Ascorbylpalmitat | 0,1 | g |
| Lecithin (Phospholipon 100®) | 0,5 | g |

37.

| | | |
|---|---|---|
| Wirkstoff B | 30 | g |
| Ethyloleat | 50 | g |
| Ascorbylpalmitat | 0,1 | g |
| Lecithin (Phospholipon 100®) | 0,5 | g |
| Benzylalkohol | 3 | g |

38.

| | | |
|---|---|---|
| Wirkstoff C | 25 | g |
| Miglyol 812® | 50 | g |
| Benzylalkohol | 3 | g |
| Lecithin (Phospholipon 100®) | 0,5 | g |
| Ascorbylpalmitat | 0,1 | g |

39.

| | | |
|---|---|---|
| Ciprofloxacin-HCl | 5,8 | g |
| Benzylalkohol | 2,0 | g |
| Sojalecithin gereinigt | 0,5 | g |
| Mittelkettige Triglyceride DAB9 ad | 100,0 | ml |

40.

| | | |
|---|---|---|
| Ciprofloxacin-HCl | 11,6 | g |
| Benzylalkohol | 2,0 | g |
| Eilecithin gereinigt | 0,5 | g |
| Mittelkettige Triglyceride DAB9 ad | 100,0 | ml |

41.

| | | |
|---|---|---|
| Ciprofloxacin-HCl | 11,6 | g |
| Benzylalkohol | 2,0 | g |
| Sojalecithin gereinigt | 5,0 | g |
| Miglyol 812® ad | 100,0 | ml |

42.

| | | |
|---|---|---|
| Ciprofloxacin-HCl | 11,6 | g |
| Ethanol absolut | 5,0 | g |
| Eilecithin gereinigt | 5,0 | g |
| Mittelkettige Triglyceride DA89 ad | 100,0 | ml |

43.

| | | |
|---|---|---|
| Ciprofloxacin-HCl | 11,6 | g |
| Ethanol absolut | 5,0 | g |
| Sojalecithin gereinigt | 5,0 | g |
| Milchsäure konzentriert | 0,05 - 1,0 | g |
| Miglyol 812® | ad 100,0 | ml |

44.

| | | |
|---|---|---|
| Ciprofloxacin-Laktat | 12,7 | g |
| Benzylalkohol | 2,0 | g |
| Sojalecithin gereinigt | 5,0 | g |
| Mittelkettige Triglyceride | ad 100,0 | ml |

45.

| | | |
|---|---|---|
| Ciprofloxacin-Mesilat | 13,42 | g |
| Ethanol | 10,0 | g |
| Eilecithin gereinigt | 10,0 | g |
| Ethyloleat | ad 100,0 | ml |

46.

| | | |
|---|---|---|
| Ciprofloxacin-Laktat | 12,7 | g |
| Ethanol | 5,0 | g |
| Sojalecithin gereinigt | 5,0 | g |
| Milchsäure konzentriert | 0,05 - 1,0 | g |
| Miglyol 812® | ad 100,0 | ml |

47.

| | | |
|---|---|---|
| Ciprofloxacin-HCl | 11,6 | g |
| Ethanol | 10,0 | g |
| Sojalecithin gereinigt | 5,0 | g |
| Methansulfonsäure | 0,05 - 1,0 | g |
| Miglyol 812® | ad 100,0 | ml |

48.

| | | |
|---|---|---|
| Ciprofloxacin-HCl | 11,6 | g |
| Ethanol | 5,0 | g |
| Solalecithin gereinigt | 5,0 | g |
| Citronensäure wasserfrei mikronisiert | 0,05 - 1,0 | g |
| Miglyol 812® | ad 100,0 | ml |

## Patentansprüche

### Patentansprüche für folgende Vertragsstaaten : AT-BE-CH-LI-DE-FR-GB-IT-NL-SE

1. i.m. Injektionsformulierungen von Gyrase-Inhibitoren, enthaltend 0,05 bis 70 Gew.-% eines Gyrase-Inhibitors der allgemeinen Formel

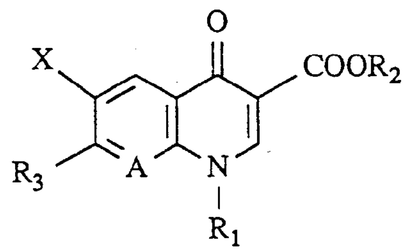

(I),

in welcher
$R^1$ für Methyl, Ethyl, Propyl, Isopropyl, Cyclopropyl, Vinyl, 2-Hydroxyethyl, 2-Fluorethyl, Methoxy, Amino, Methylamino, Dimethylamino, Ethylamino, Phenyl, 4-Fluorphenyl, 2,4-Difluorphenyl,
$R^2$ für Wasserstoff, Alkyl mit 1 bis 4 Kohlenstoffatomen, (5-Methyl-2-oxo-1,3-dioxol-4-yl)-methyl,
$R^3$ für Methyl oder eine cyclische Aminogruppe wie

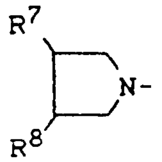, 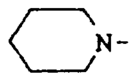, 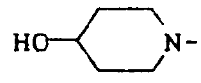, 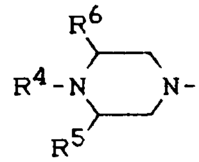,

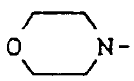, 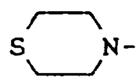, 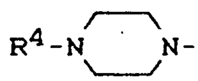, 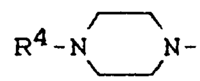,

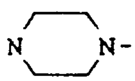, 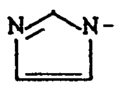, 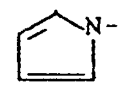

steht, worin

$R^4$ für Wasserstoff, Alkyl mit 1 bis 4 Kohlenstoffatomen, 2-Hydroxyethyl, Allyl, Propargyl, 2-Oxopropyl, 3-Oxobutyl, Phenacyl, Formyl, $CFCl_2$-S-, $CFCl_2$-$SO_2$-, $CH_3O$-CO-S-, Benzyl, 4-Aminobenzyl,

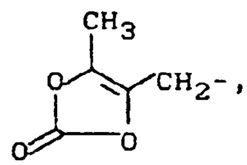,

$R^5$ für Wasserstoff, Methyl,
$R^6$ für Wasserstoff, Alkyl mit 1 bis 4 Kohlenstoffatomen, Phenyl, Benzyloxymethyl,
$R^7$ für Wasserstoff, Amino, Methylamino, Ethylamino, Aminomethyl, Methylaminomethyl, Ethylaminomethyl, Dimethylaminomethyl, Hydroxy, Hydroxymethyl,
$R^8$ für Wasserstoff, Methyl, Ethyl oder Chlor steht,
X für Fluor, Chlor oder Nitro und
A für N oder C-$R^6$ steht, worin
$R^6$ für Wasserstoff, Halogen wie Fluor oder Chlor, Methyl oder Nitro steht oder auch gemeinsam mit $R^1$ eine Brücke der Struktur

$$-O-CH_2-\underset{|}{C}H-CH_3, \quad -S-CH_2-\underset{|}{C}H-CH_3 \text{ oder}$$

$$-CH_2-CH_2-\underset{|}{C}H-CH_3$$

bilden kann, gegebenenfalls als Salze mit einer Säure oder Base in öliger Suspension.

2. i.m. Injektionsformulierungen nach Anspruch 1, dadurch gekennzeichnet, daß die Partikelgröße der Gyrase-Inhibitoren 0,5-150 µm, bevorzugt 4 bis 40 µm beträgt.

3. i.m. Injektionsformulierungen nach den Ansprüchen 1 und 2, dadurch gekennzeichnet, daß sie 2,5 bis 50 Gew.-% Gyrase-Inhibitor enthalten.

4. Verfahren zur Herstellung von i.m. Injektionsformulierungen gemäß Anspruch 1, dadurch gekennzeichnet, daß man die Wirkstoffe der allgemeinen Formel I nach Anspruch 1 in mikronisierter Form in ein vorgelegtes Öl, das gegebenenfalls bereits Antioxidatien, Stabilisatoren und oberflächenaktive Substanzen enthält, gegebennenfalls unter Stickstoffbegasung einarbeitet und diese Formulierung gegebenenfalls nach bekannten Methoden nachhomogenisiert.

5. i.m. Injektionsformen nach Anspruch 1 zur Anwendung in einem Verfahren zur therapeutischen Behandlung des menschlichen oder tierischen Körpers.

6. Intramuskuläre Injektionsformen nach Anspruch 1 auf öliger Basis, die die Wirksubstanz in wasserlöslicher, kristalliner oder amorpher Form enthalten in Form des Hydrochlorids, Laktates, Mesilates, p-Tolylsulfonates und Salzen anderer physiologisch verträglicher Säuren.

7. Intramuskuläre Injektionsformen nach Anspruch 1 auf öiger Basis, die grenzflächenaktive Substanzen wie Lecithin in Form von Soja-, Ei-, Cerebral- oder Raps-Lecithin oder andere physiologisch verträgliche Tenside in Konzentrationen von 0,1-30 % G/V enthalten.

8. Intramuskuläre Injektionsformen nach Anspruch 7 auf öliger Basis, die grenzflächenaktive Substanzen wie Lecithin in Form von Soja-, Ei-, Cerebral- oder Raps-Lecithin oder andere physiologisch verträgliche Tenside in Konzentrationen von 0,2-10 % G/V enthalten.

9. Intramuskuläre Injektionsformen nach Anspruch 7 auf öiger Basis, die grenzflächenaktive Substanzen wie Lecithin in Form von Soja-, Ei-, Cerebral- oder Raps-Lecithin oder andere physiologisch verträgliche Tenside in Konzentrationen von 0,5-5 % G/V enthalten.

10. Intramuskuläre Injektionsformen nach Anspruch 1 auf öiger Basis, die neben den wasserlöslichen, in Form ihrer Salze eingesetzten Wirkstoffen einen Überschuß an physiologisch verträglicher Säure im Bereich von 1-300 mmol/l enthalten.

11. Intramuskuläre Injektionsformen nach Anspruch 10 auf öiger Basis, die neben den wasserlöslichen, in Form ihrer Salze eingesetzten Wirkstoffen einen Überschuß an physiologisch verträglicher Säure im Bereich von 5-50 mmol/l enthalten.

12. Intramuskuläre Injektionsformen nach Anspruch 10 auf öiger Basis, die neben den wasserlöslichen, in Form ihrer Salze eingesetzten Wirkstoffen einen Überschuß an physiologisch verträglicher Säure im Bereich von 10-30 mmol/l enthalten.

13. Intramuskuläre Injektionsformen nach den Ansprüchen 1.0, 11 und 12, dadurch gekennzeichnet, daß es sich bei der Säure um Milchsäure oder Citronensäure handelt.

**Patentansprüche für folgende Vertragsstaaten : ES und GR**

1. Verfahren zur Herstellung von i.m. Injektionsformulierungen von Gyrase-Inhibitoren, enthaltend 0,05 bis 70 Gew.-% eines Gyrase-Inhibitors der allgemeinen Formel

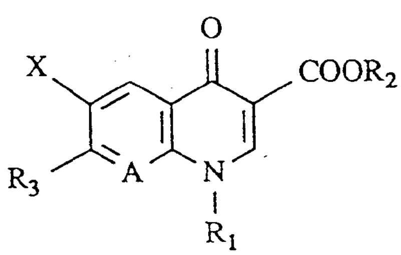

(I),

in welcher

$R^1$ für Methyl, Ethyl, Propyl, Isopropyl, Cyclopropyl, Vinyl, 2-Hydroxyethyl, 2-Fluorethyl, Methoxy, Amino, Methylamino, Dimethylamino, Ethylamino, Phenyl, 4-Fluorphenyl, 2,4-Difluorphenyl,

$R^2$ für Wasserstoff, Alkyl mit 1 bis 4 Kohlenstoffatomen, (5-Methyl-2-oxo-1,3-dioxol-4-yl)-methyl,

$R^3$ für Methyl oder eine cyclische Aminogruppe wie

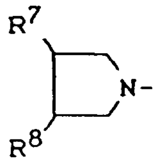, 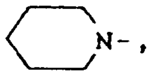, 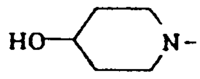, 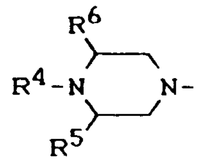,

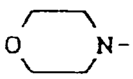, 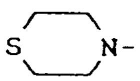, 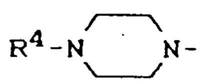, 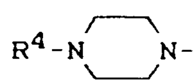,

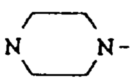, 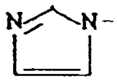, 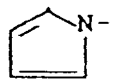

steht, worin
$R^4$ für Wasserstoff, Alkyl mit 1 bis 4 Kohlenstoffatomen, 2-Hydroxyethyl, Allyl, Propargyl, 2-Oxopropyl, 3-Oxobutyl, Phenacyl, Formyl, $CFCl_2$-S-, $CFCl_2$-$SO_2$-, $CH_3O$-CO-S-, Benzyl, 4-Aminobenzyl,

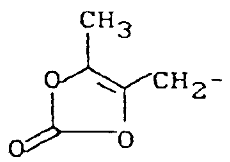,

$R^5$ für Wasserstoff, Methyl,
$R^6$ für Wasserstoff, Alkyl mit 1 bis 4 Kohlenstoffatomen, Phenyl, Benzyloxymethyl,
$R^7$ für Wasserstoff, Amino, Methylamino, Ethylamino, Aminomethyl, Methylaminomethyl, Ethylaminomethyl, Dimethylaminomethyl, Hydroxy, Hydroxymethyl,
$R^8$ für Wasserstoff, Methyl, Ethyl oder Chlor steht,
X für Fluor, Chlor oder Nitro und A für N oder C-$R^6$ steht, worin
$R^6$ für Wasserstoff, Halogen wie Fluor oder Chlor, Methyl oder Nitro steht oder auch gemeinsam mit $R^1$ eine Brücke der Struktur

$-O-CH_2-CH(-)-CH_3$, $-S-CH_2-CH(-)-CH_3$ oder

$-CH_2-CH_2-CH(-)-CH_3$

bilden kann, dadurch gekennzeichnet, daß man die Wirkstoffe der allgemeinen Formel I nach Anspruch 1 in mikronisierter Form in ein vorgelegtes Öl, das gegebenenfalls bereits Antioxidatien, Stabilisatoren und oberflächenaktive Substanzen enthält, gegebennenfalls unter Stickstoffbegasung einarbeitet und diese Formulierung gegebenenfalls nach bekannten Methoden nachhomogenisiert.

2. Verfahren nach Anspruch 1, dadurch gekennzeichnet, daß die Partikelgröße der Gyrase-Inhibitoren 0,5-150 μm, bevorzugt 4 bis 40 μm beträgt.

3. Verfahren zur Herstellung von i.m. Injektionsformulierungen nach den Ansprüchen 1 und 2, dadurch gekennzeichnet, daß sie 2,5 bis 50 Gew.-% Gyrase-Inhibitor enthalten.

4. Verfahren nach Anspruch 1, dadurch gekennzeichnet, daß die Wirksubstanz in wasserlöslicher, kristalliner oder amorpher Form als Hydrochlorid, Laktat, Mesilat, p-Tolylsulfonat oder Salz anderer physiologisch verträglicher Säuren eingesetzt wird.

5. Verfahren nach Anspruch 1, dadurch gekennzeichnet, daß grenzflächenaktiveSubstanzen wie Lecithin in Form von Soja-, Ei-, Cerebral- oder Raps-Lecithin oder andere physiologisch verträgliche Tenside in Konzentrationen von 0,1 - 30 % G/V eingesetzt werden.

6. Verfahren nach Anspruch 5, dadurch gekennzeichnet, daß grenzflächenaktiveSubstanzen wie Lecithin in Form von Soja-, Ei-, Cerebral- oder Raps-Lecithin oder andere physiologisch verträgliche Tenside in Konzentrationen von 0,2 - 10 % G/V eingesetzt werden.

7. Verfahren nach Anspruch 1, dadurch gekennzeichnet, daß neben den wasserlöslichen, in Form ihrer Salze eingesetzten Wirkstoffen ein Überschub an physiologisch verträglicher Säure im Bereich von 1 -300 mmol/l eingesetzt wird.

8. Verfahren nach Anspruch 7, dadurch gekennzeichnet, daß neben den wasserlöslichen, in Form ihrer Salze eingesetzten Wirkstoffen ein Überschub an physiologisch verträglicher Säure im Bereich von 5-50 mmol/l eingesetzt wird.

9. Verfahren nach Ansprüchen 7 und 8, dadurch gekennzeichnet, daß es sich bei der Säure um Milchsäure oder Citronensäure handelt.

## Claims

### Claims for the following Contracting States : AT-BE-CH-LI-DE-FR-GB-IT-NL-SE

1. I.m. injection formulations of gyrase inhibitors, containing 0.05 to 70% by weight of a gyrase inhibitor of the general formula

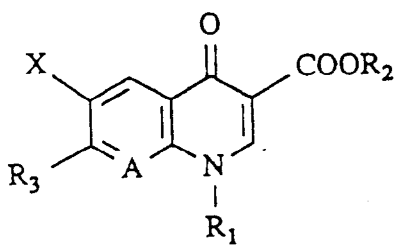

(I)

in which
$R^1$ represents methyl, ethyl, propyl, isopropyl, cyclopropyl, vinyl, 2-hydroxyethyl, 2-fluoroethyl, methoxy, amino, methylamino, dimethylamino, ethylamino, phenyl, 4-fluorophenyl or 2,4-difluorophenyl,
$R^2$ represents hydrogen, alkyl having 1 to 4 carbon atoms or(5-methyl-2-oxo-1,3-dioxol-4-yl)-methyl,
$R^3$ represents methyl or a cyclic amino group, such as

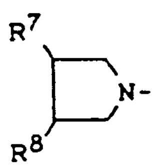 , 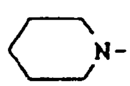 , 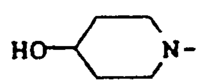 , 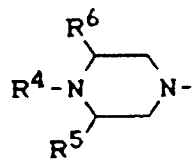 ,

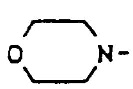 , 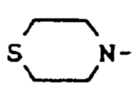 , 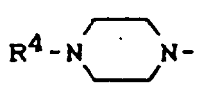 , 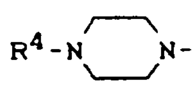 ,

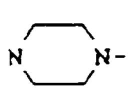 , 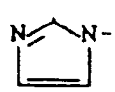 , 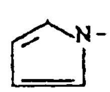

wherein
$R^4$ represents hydrogen, alkyl having 1 to 4 carbon atoms, 2-hydroxyethyl, allyl, propargyl, 2-oxopropyl, 3-oxobutyl, phenacyl, formyl, $CFCl_2$-S-, $CFCl_2$-$SO_2$-, $CH_2$O-CO-S-, benzyl, 4-aminobenzyl or

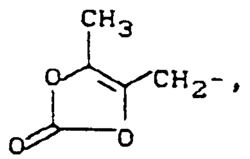

$R^5$ represents hydrogen or methyl
$R^6$ represents hydrogen, alkyl having 1 to 4 carbon atoms, phenyl or benzyloxymethyl,
$R^7$ represents hydrogen, amino, methylamino, ethylamino, aminomethyl, methylaminomethyl, ethylaminomethyl, dimethylaminomethyl, hydroxyl or hydroxymethyl,
$R^8$ represents hydrogen, methyl, ethyl or chlorine,
X represents fluorine, chlorine or nitro and A represents N or C-$R^6$, wherein

$R^6$ represents hydrogen, halogen, such as fluorine or chlorine, methyl or nitro or, together with $R^1$, can also form a bridge having the structure

$-O-CH_2-CH-CH_3$, $-S-CH_2-CH-CH_3$ or

$-CH_2-CH_2-CH-CH_3$

if appropriate as a salt with an acid or base in oily suspension.

2. I.m. injection formulations according to Claim 1, characterised in that the particle size of the gyrase inhibitors is 0.5-150 µm, preferably 4 to 40 µm.

3. I.m. injection formulations according to Claims 1 and 2, characterized in that they contain 2.5 to 50% by weight of gyrase inhibitor.

4. Process for the preparation of i.m. injection formulations according to Claim 1, characterized in that the active compounds of the general formula I according to Claim 1 are incorporated in micronized form, if appropriate while gassing with nitrogen, into an oil which is initially introduced into the vessel and if appropriate already contains antioxidants, stabilizers and surface-active substances, and if appropriate this formulation is after-homogenized by known methods.

5. I.m. injection forms according to Claim 1 for use in a method for the therapeutic treatment of the human or animal body.

6. Oil-based intramuscular injection forms according to Claim 1, which contain the active substance in water-soluble, crystalline or amorphous form in the form of the hydrochloride, lactate, mesylate, p-toluenesulphonate or salts of other physiologically tolerable acids.

7. Oil-based intramuscular injection forms according to Claim 1, which contain surface-active substances such as lecithin in the form of soya bean, egg, cerebral or rape-seed lecithin, or other physiologically tolerable surfactants, in concentrations of 0.1-30% w/v.

8. Oil-based intramuscular injection forms according to Claim 7, which contain surface-active substances such as lecithin in the form of soya bean, egg, cerebral or rape-seed lecithin, or other physiologically tolerable surfactants, in concentrations of 0.2-10% w/v.

9. Oil-based intramuscular injection forms according to Claim 7, which contain surface-active substances such as lecithin in the form of soya bean, egg, cerebral or rape-seed lecithin, or other physiologically tolerable surfactants, in concentrations of 0.5-5% w/v.

10. Oil-based intramuscular injection forms according to Claim 1, which contain, in addition to the water-soluble active compounds employed in the form of their salts, an excess of a physiologically tolerable acid in the range from 1-300 mmol/l.

11. Oil-based intramuscular injection forms according to Claim 10, which contain, in addition to the water-soluble active compounds employed in the form of their salts, an excess of a physiologically tolerable acid in the range from 5-50 mmol/l.

12. Oil-based intramuscular injection forms according to Claim 10, which contain, in addition to the water-soluble active compounds employed in the form of their salts, an excess of a physiologically tolerable acid in the range from 10-30 mmol/l.

13. Intramuscular injection forms according to Claims 10, 11 and 12, characterized in that the acid is lactic acid or citric acid.

**Claims for the following Contracting States : ES and GR**

1. Process for the preparation of i.m. injection formulations of gyrase inhibitors, containing 0.05 to 70% by weight of a gyrase inhibitor of the general formula.

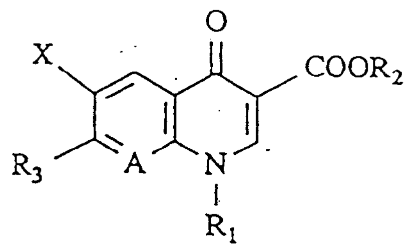

(I)

in which
$R^1$ represents methyl, ethyl, propyl, isopropyl, cyclopropyl, vinyl, 2-hydroxyethyl, 2-fluoroethyl, methoxy, amino, methylamino, dimethylamino, ethylamino, phenyl, 4-fluorophenyl or 2,4-difluorophenyl,
$R^2$ represents hydrogen, alkyl having 1 to 4 carbon atoms or(5-methyl-2-oxo-1,3-dioxol-4-yl)-methyl,
$R^3$ represents methyl or a cyclic amino group, such as

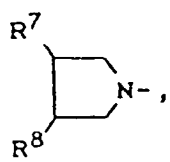 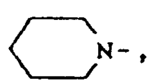 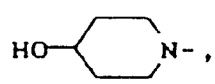 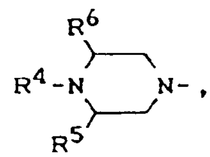

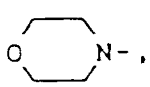 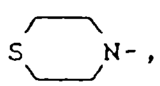 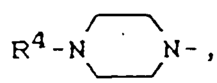 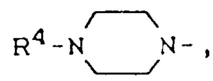

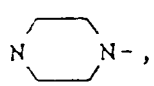 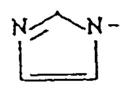 , 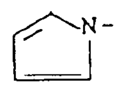

wherein
$R^4$ represents hydrogen, alkyl having 1 to 4 carbon atoms, 2-hydroxyethyl, allyl, propargyl, 2-oxopropyl, 3-oxobutyl, phenacyl, formyl, $CFCl_2$-S-, $CFCl_2$-$SO_2$-, $CH_2O$-CO-S-, benzyl, 4-aminobenzyl or

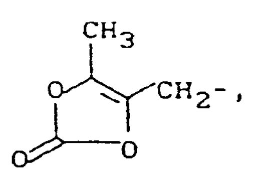

$R^5$ represents hydrogen or methyl
$R^6$ represents hydrogen, alkyl having 1 to 4 carbon atoms, phenyl or benzyloxymethyl,
$R^7$ represents hydrogen, amino, methylamino, ethylamino, aminomethyl, methylaminomethyl, ethylaminomethyl, dimethylaminomethyl, hydroxyl or hydroxymethyl,
$R^8$ represents hydrogen, methyl, ethyl or chlorine,
X represents fluorine, chlorine or nitro and A represents N or C-$R^6$, wherein

$R^6$ represents hydrogen, halogen, such as fluorine or chlorine, methyl or nitro or, together with $R^1$, can also form a bridge having the structure

$-O-CH_2-CH-CH_3$, $-S-CH_2-CH-CH_3$ or

$-CH_2-CH_2-CH-CH_3$

characterized in that the active compounds of the general formula I according to Claim 1 are incorporated in micronized form, if appropriate while gassing with nitrogen, into an oil which is initially introduced into the vessel and if appropriate already contains antioxidants, stabilizers and surface-active substances, and if appropriate this formulation is after-homogenized by known methods.

2. Process according to Claim 1, characterized in that the particle size of the gyrase inhibitors is 0.5-150 μm, preferably 4 to 40 μm.

3. Process for the preparation of i.m. injection formulations according to Claims 1 and 2, characterized in that they contain 2.5 to 50% by weight of gyrase inhibitor.

4. Process according to Claim 1, characterized in that the active substance is employed in water-soluble, crystalline or amorphous form as hydrochloride, lactate, mesylate, p-toluenesulphonate or salt of another physiologically tolerable acid.

5. Process according to Claim 1, characterized in that surface-active substances such as lecithin in the form of soya bean, egg, cerebral or rape-seed lecithin, or other physiologically tolerable surfactants, are employed in concentrations of 0.1-30% w/v.

6. Process according to Claim 5, characterized in that surface-active substances such as lecithin in the form of soya bean, egg, cerebral or rape-seed lecithin, or other physiologically tolerable surfactants, are employed in concentrations of 0.2-10% w/v.

7. Process according to Claim 1, characterized in that, in addition to the water-soluble active compounds employed in the form of their salts, an excess of a physiologically tolerable acid in the range from 1-300 mmol/l is employed.

8. Process according to Claim 7, characterized in that, in addition to the water-soluble active compounds employed in the form of their salts, an excess of a physiologically tolerable acid in the range from 5-50 mmol/l is employed.

9. Process according to Claims 7 and 8, characterized in that the acid is lactic acid or citric acid.

## Revendications

### Revendications pour les Etats contractants suivants : AT-BE-CH-LI-DE-FR-GB-IT-NL-SE

1. Formulations d'inhibiteurs de la gyrase pour injections intramusculaires, contenant de 0,05 à 70% en poids d'un inhibiteur de la gyrase de formule générale :

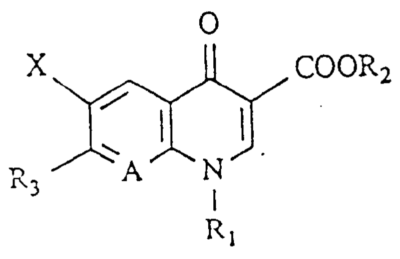

(I),

dans laquelle

$R^1$ représente un groupe méthyle, éthyle, propyle, isopropyle, cyclopropyle, vinyle, 2-hydroxyéthyle, 2-fluoréthyle, méthoxy, amino, méthylamino, diméthylamino, éthylamino, phényle, 4-fluorophényle, 2,4-difluorophényle,

$R^2$ représente l'hydrogène, un groupe alkyle en $C_1$-$C_4$, un groupe (5-méthyl-2-oxo-1,3-dioxole-4-yl)-méthyle,

$R^3$ représente un groupe méthyle ou un groupe amino cyclique tel que

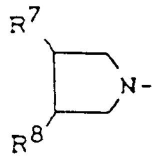 , 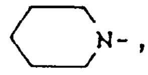 , 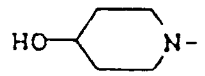 , 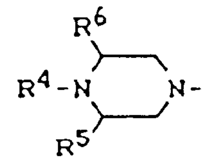 ,

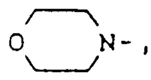 , 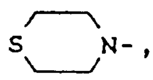 , 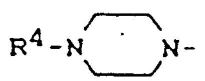 , 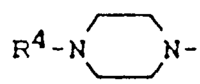 ,

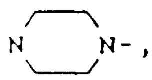 , 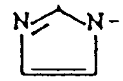 , 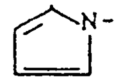

dans lesquels

$R^4$ représente l'hydrogène, un groupe alkyle en $C_1$-$C_4$, 2-hydroxyéthyle, allyle, propargyle, 2-oxopropyle, 3-oxobutyle, phénacyle, formyle, $CFCl_2$-S-, $CFCl_2$-$SO_2$-, $CH_3O$-CO-S-, benzyle, 4-aminobenzyle,

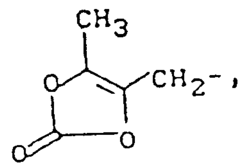 ,

$R^5$ représente l'hydrogène, un groupe méthyle,

$R^6$ représente l'hydrogène, un groupe alkyle en $C_1$-$C_4$, phényle, benzyloxyméthyle,

$R^7$ représente l'hydrogène, un groupe amino, méthylamino, éthylamino, aminométhyle, méthylaminométhyle, éthylaminométhyle, diméthylaminométhyle, hydroxy, hydroxyméthyle,

$R^8$ représente l'hydrogène, un groupe méthyle, éthyle ou le chlore,

X représente le fluor, le chlore ou un groupe nitro, et

A représente N ou C-$R^6$ dans lequel

$R^6$ représente l'hydrogène, un halogène tel que le fluor, le chlore, un groupe méthyle ou nitro ou forme avec $R^1$ un pont de structure

$$-O-CH_2-\underset{|}{C}H-CH_3, \quad -S-CH_2-\underset{|}{C}H-CH_3 \text{ ou}$$

$$-CH_2-CH_2-\underset{|}{C}H-CH_3$$

éventuellement à l'état de sels d'acides ou de bases, en suspension huileuse.

2. Formulations pour injections intramusculaires selon revendication 1, caractérisées en ce que la dimension de particule des inhibiteurs de la gyrase est de 0,5 à 150 µm, de préférence de 4 à 40 µm.

3. Formulations pour injections intramusculaires selon les revendications 1 et 2, caractérisées en ce qu'elles contiennent de 2,5 à 50% en poids d'inhibiteur de la gyrase.

4. Procédé de préparation des formulations pour injections intramusculaires de la revendication 1, caractérisé en ce que l'on incorpore, éventuellement en atmosphère d'azote, les substances actives de formule générale I de la revendication 1, à l'état micronisé, dans une huile contenant le cas échéant déjà des antioxydants, des stabilisants, et des agents tensioactifs, et le cas échéant on soumet la composition à une homogénéisation complémentaire selon des techniques connues.

5. Formes pour injections intramusculaires selon la revendication 1, pour l'utilisation dans un procédé pour le traitement thérapeutique de l'organisme humain ou animal.

6. Formes d'injections intramusculaires selon la revendication 1, à base huileuse, contenant la substance active à l'état soluble dans l'eau, cristallisée ou amorphe, sous la forme du chlorhydrate, du lactate, du mésilate, du p-tolylsulfonate ou d'un sel d'un autre acide acceptable pour l'usage pharmaceutique.

7. Formes d'injections intramusculaires selon la revendication 1, à base huileuse, contenant des agents tensioactifs tels que la lécithine à l'état de lécithine de soja, d'oeufs, de cervelles ou de colza ou d'autres agents tensioactifs acceptables pour l'usage pharmaceutique, à des concentrations de 0,1 à 30% en poids/ volume.

8. Formes d'injections intramusculaires selon la revendication 7, à base huileuse, contenant des agents tensioactifs tels que la lécithine sous la forme de lécithine de soja, d'oeufs, de cervelles, de colza, ou d'autres agents tensioactifs acceptables pour l'usage pharmaceutique, à des concentrations de 0,2 à 10% en poids/volume.

9. Formes d'injections intramusculaires selon la revendication 7, à base huileuse, contenant des agents tensioactifs tels que la lécithine sous la forme de lécithine de soja, d'oeufs, de cervelles ou de colza, ou d'autres agents tensioactifs acceptables pour l'usage pharmaceutique, à des concentrations de 0,5 à 5% en poids/volume.

10. Formes d'injections intramusculaires selon la revendication 1, à base huileuse contenant, en plus des substances actives solubles dans l'eau, mises en oeuvre à l'état de sels, un excès d'acide acceptable pour l'usage pharmaceutique se situant dans l'intervalle de 1 à 300 mmol/l.

11. Formes d'injections intramusculaires selon la revendication 10, à base huileuse, contenant, en plus des substances actives solubles dans l'eau, mises en oeuvre à l'état de sels, un excès d'acide acceptable pour l'usage pharmaceutique se situant dans l'intervalle de 5 à 50 mmol/l.

12. Formes d'injections intramusculaires selon la revendication 10, à base huileuse, contenant en plus des substances actives solubles dans l'eau, mises en oeuvre à l'état de sels, un excès d'acide acceptable pour l'usage pharmaceutique se situant dans l'intervalle de 10 à 30 mmol/l.

13. Formes d'injections intramusculaires selon les revendications 10, 11 et 12, caractérisées en ce que l'acide est l'acide lactique ou l'acide citrique.

**Revendications pour les Etats contractants suivants : ES et GR**

1. Procédé de préparation de formulations pour injections intramusculaires d'inhibiteurs de la gyrase, contenant de 0,05 à 70% en poids d'un inhibiteur de la gyrase de formule générale :

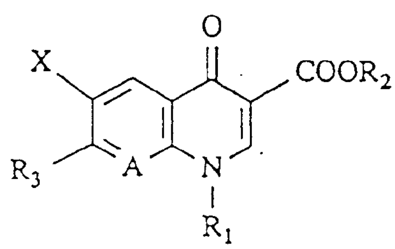

(I),

dans laquelle

$R^1$ représente un groupe méthyle, éthyle, propyle, isopropyle, cyclopropyle, vinyle, 2-hydroxyéthyle, 2-fluoréthyle, méthoxy, amino, méthylamino, diméthylamino, éthylamino, phényle, 4-fluorophényle, 2,4-difluorophényle,

$R^2$ représente l'hydrogène, un groupe alkyle en $C_1$-$C_4$, un groupe (5-méthyl-2-oxo-1,3-dioxole-4-yl)-méthyle,

$R^3$ représente un groupe méthyle ou un groupe amino cyclique tel que

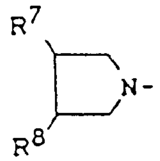 , 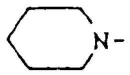 , 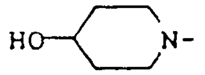 , 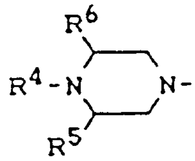 ,

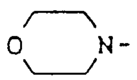 , 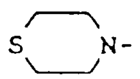 , 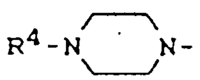 , 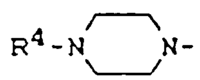 ,

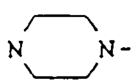 , 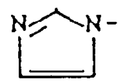 , 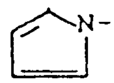

dans lesquels
$R^4$ représente l'hydrogéne, un groupe alkyle en $C_1$-$C_4$, 2-hydroxyéthyle, allyle, propargyle, 2-oxopropyle, 3-oxobutyle, phénacyle, formyle, $CFCl_2$-S-, $CFCl_2$-$SO_2$-, $CH_3O$-CO-S-, benzyle, 4-aminobenzyle,

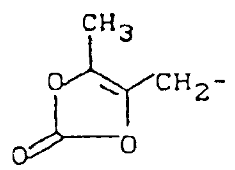

$R^5$ représente l'hydrogène, un groupe méthyle,
$R^6$ représente l'hydrogène, un groupe alkyle en $C_1$-$C_4$, phényle, benzyloxyméthyle,
$R^7$ représente l'hydrogène, un groupe amino, méthylamino, éthylamino, aminométhyle, méthylaminométhyle, éthylaminométhyle, diméthylaminométhyle, hydroxy, hydroxyméthyle,
$R^8$ représente l'hydrogéne, un groupe méthyle, éthyle ou le chlore,
X représente le fluor, le chlore ou un groupe nitro, et
A représente N ou C-$R^6$ dans lequel
$R^6$ représente l'hydrogène, un halogène tel que le fluor ou le chlore, un groupe méthyle ou nitro ou bien, avec $R^1$, un pont de structure

$$-O-CH_2-CH-CH_3, \quad -S-CH_2-CH-CH_3 \text{ ou}$$

$$-CH_2-CH_2-CH-CH_3$$

caractérisé en ce que l'on incorpore, éventuellement en atmosphère d'azote, les substances actives de formule générale I de la revendication 1, à l'état micronisé, dans une huile contenant le cas échéant déjà des antioxydants, des stabilisants et des agents tensioactifs, puis le cas échéant on soumet la formulation à une homogénéisation complémentaire par des modes opératoires connus.

2. Procédé selon la revendication 1, caractérisé en ce que la dimension de particule des inhibiteurs de la gyrase est de 0,5 à 150 μm, de préférence de 4 à 40 μm.

3. Procédé de préparation de formulations pour injections intramusculaires selon les revendications 1 et 2, caractérisé en ce qu'elles contiennent de 2,5 à 50% en poids d'inhibiteur de la gyrase.

4. Procédé selon la revendication 1, caractérisé en ce que la substance active est mise en oeuvre sous une forme soluble dans l'eau, cristallisée ou amorphe, à l'état de chlorhydrate, de lactate, de mésilate, de p-tolylsulfonate ou de sel d'un autre acide acceptable pour l'usage pharmaceutique.

5. Procédé selon la revendication 1, caractérisé en ce que les agents tensioactifs, tels que la lécithine sous forme de lécithine de soja, d'oeufs, de cervelles ou de colza, ou d'autres agents tensioactifs acceptables pour l'usage pharmaceutique, sont mis en oeuvre à des concentrations de 0,1 à 30% en poids/volume.

6. Procédé selon la revendication 5, caractérisé en ce que les agents tensioactifs tels que la lécithine sous

la forme de lécithine de soja, d'oeufs, de cervelles ou de colza, ou d'autres agents tensioactifs acceptables pour l'usage pharmaceutique, sont mis en oeuvre à des concentrations de 0,2 à 10% en poids/volume.

7. Procédé selon la revendication 1, caractérisé en ce que, en plus des substances actives solubles dans l'eau, mises en oeuvre à l'état de sels, on utilise un excès d'acide acceptable pour l'usage pharmaceutique se situant dans l'intervalle de 1 à 300 mmol/l.

8. Procédé selon la revendication 7, caractérisé en ce que, en plus des substances actives solubles dans l'eau, mises en oeuvre à l'état de sels, on utilise un excès d'acide acceptable pour l'usage pharmaceutique se situant dans l'intervalle de 5 à 50 mmol/l.

9. Procédé selon les revendications 7 et 8, caractérisé en ce que l'acide utilisé est l'acide lactique ou l'acide citrique.

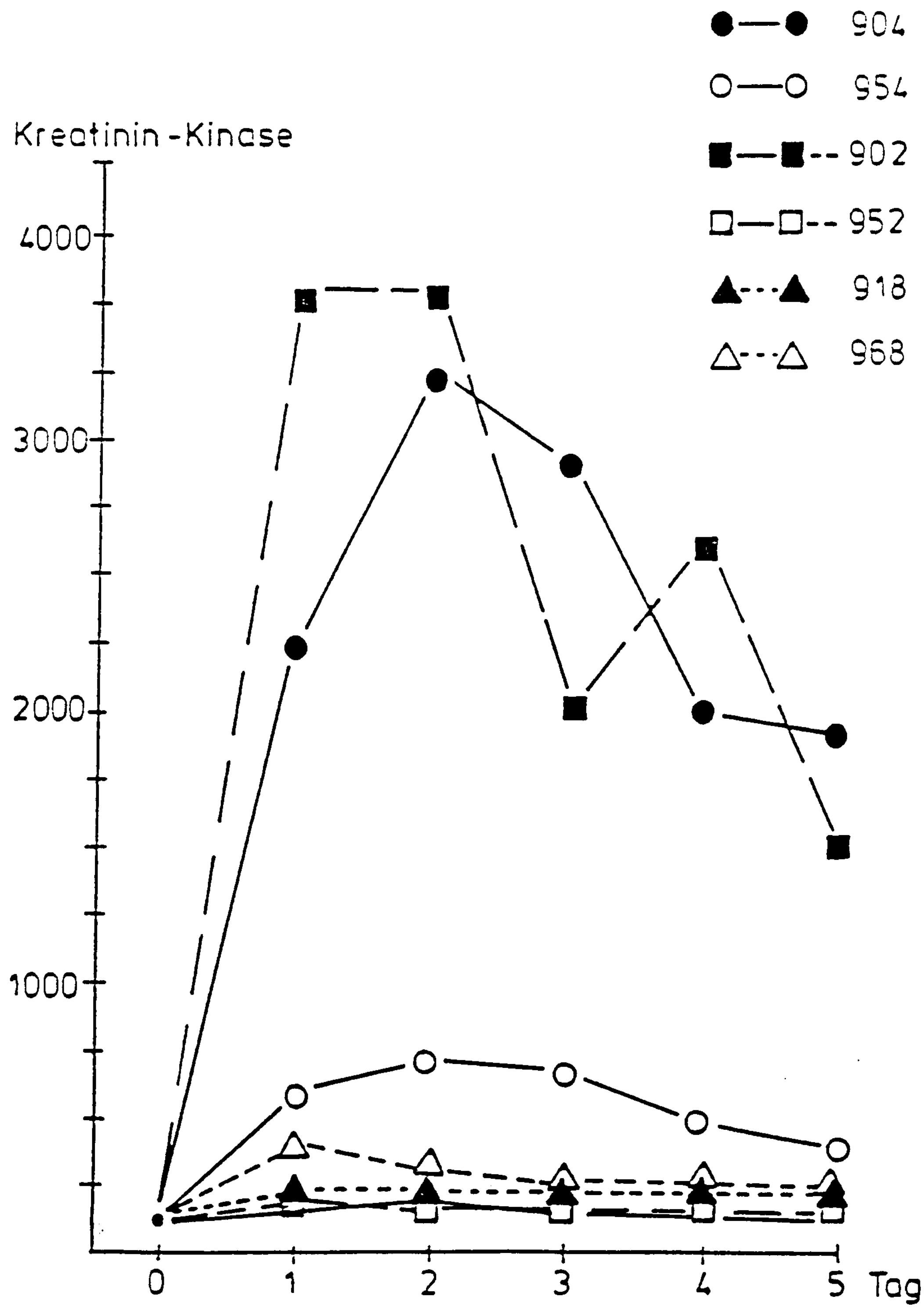
Kreatinin-Kinase
4000
3000
2000
1000
0
1
2
3
4
5
Tag
904
954
902
952
918
968

FIG.1

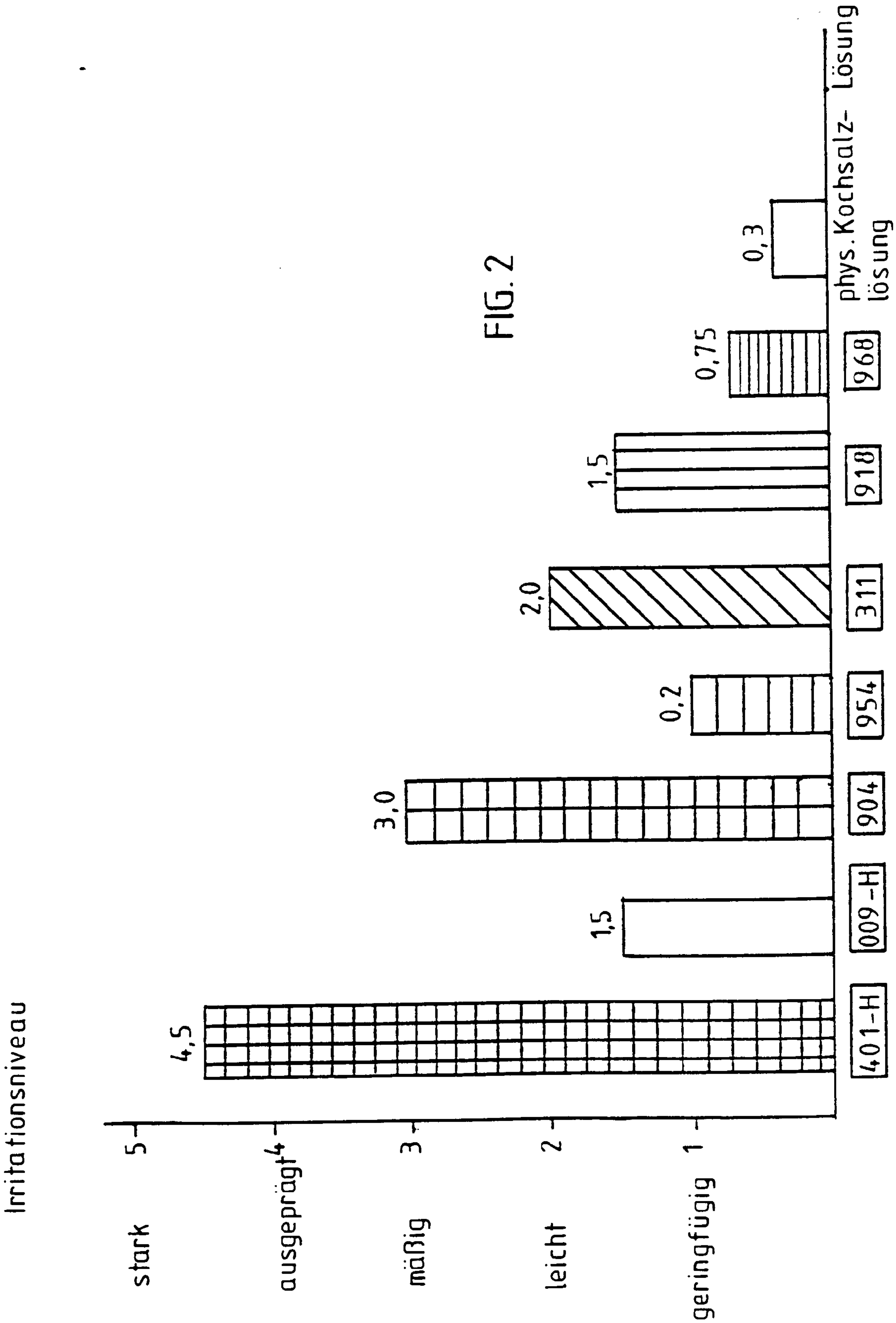
Irritationsniveau
stark
ausgeprägt
mäßig
leicht
geringfügig
5
4
3
2
1
4,5
4,01-H
1,5
009-H
3,0
904
0,2
954
2,0
311
1,5
918
0,75
968
0,3
phys. Kochsalz-
lösung
Lösung

FIG. 2

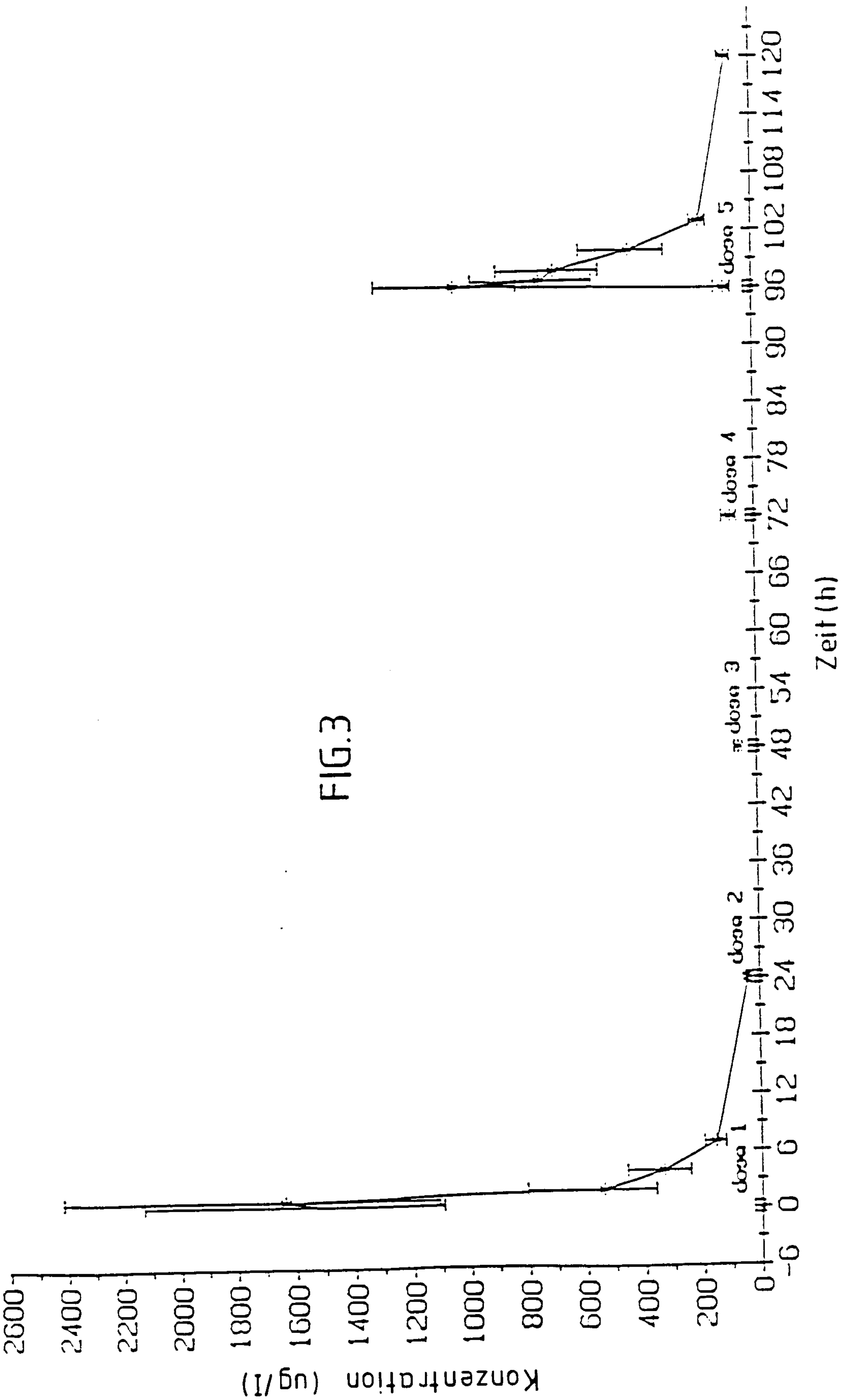
Konzentration (ug/l)
2600
2400
2200
2000
1800
1600
1400
1200
1000
800
600
400
200
0
Zeit (h)
-6
0
6
12
18
24
30
36
42
48
54
60
66
72
78
84
90
96
102
108
114
120
dosa 1
dosa 2
dosa 3
dosa 4
dosa 5

FIG.3